# NEW EUROPEAN PATENT SPECIFICATION

(11) **EP 0 701 623 B2**
(45) Date of publication and mention of the opposition decision: **04.12.2002**
(45) Mention of the grant of the patent: 14.05.1997
(21) Application number: 94918850.2
(22) Date of filing: 08.06.1994
(51) Int. Cl.: C12P 19/26

(54) **PROCESS FOR THE PREPARATION OF N-ACETYLNEURAMINIC ACID**
VERFAHREN ZUR HERSTELLUNG VON N-ACETYLNEURAMINSÄURE
PROCEDE DE PREPARATION D'ACIDE N-ACETYLNEURAMINIQUE

(30) Priority: 09.06.1993 GB 9311873
(43) Date of publication of application: 20.03.1996
(73) Proprietor: GLAXO GROUP LIMITED, Greenford, Middlesex UB6 0NN (GB)
(72) Inventor: DAWSON, Michael John, Greenford, Middlesex UB6 0HE (GB); NOBLE, David, Greenford, Middlesex UB6 0HE (GB); MAHMOUDIAN, Mahmoud, Greenford, Middlesex UB6 0HE (GB)
(74) Representative: Quillin, Helen Kaye
(86) International application number: EP9401860
(87) International publication number: WO94029476

(56) References cited:
- EP-A- 0 428 947
- WO-A-93/15214
- Dissertation Udo Kragl, Jül-2583, Februar Dissertation Udo Kragl, Jül-2583, Februar 1992, ISSN 0366-0855 1992, ISSN 0366-0855
- Justus Liebigs Ann. Chem. 1962, 659, Justus Liebigs Ann. Chem. 1962, 659, 156-163 156-163
- Methods in Enzymology, Volume L, Part C; Methods in Enzymology, Volume L, Part C; Victor Ginsburg Edition, Academic Press Victor Ginsburg Edition, Academic Press 1978, pp 70 1978, pp 70
- "Grundoperationen chemischer "Grundoperationen chemischer Verfahrenstechnik", Vauck/Müller, VCH, ISBN Verfahrenstechnik", Vauck/Müller, VCH, ISBN 3-527-28031-6 3-527-28031-6

## Description

The present invention relates to the preparation of N-acetyl-D-neuraminic acid.

N-Acetyl-D-neuraminic acid (NANA) is a sialic acid which occurs naturally as a component of complex mucoid substances such as mucolipids and mucoproteins and also as a component of oligosaccharides found for example in milk.

NANA is a useful starting material for compounds of interest as therapeutic agents. However, NANA is currently available only in limited quantities and there is a need for processes for its preparation from simple, widely available starting materials. The present invention relates to a method for the preparation of NANA from N-acetyl-D-glucosamine (NAG), and pyruvic acid.

International Patent Application No. 93/15214 (published 5th August, 1993) discloses a one-pot procedure for the preparation of NANA by treatment of NAG with pyruvic acid and NANA lyase under alkaline conditions.

European Patent Application No. 0428947 discloses a one-pot procedure for the preparation of NANA by treatment of NAG with NAG-2-epimerase, NANA lyase and pyruvate.

The present invention provides in a first aspect a process for the preparation of NANA comprising the steps of :-
1. converting NAG to N-acetyl-D-mannosamine (NAM) to yield an equilibrium mixture of NAG/NAM;
2. selectively removing NAG from the equilibrium mixture;
3. reacting NAM with pyruvate in the presence of NANA aldolase; and
4. isolating the thus-produced NANA, by crystallisation from the reaction mixture;
wherein the pyruvate : NAM starting molar ratio for step 3 is 1·5:1 to 2·5:1 and the concentration of NANA in the final reaction mixture is about 150g/litre.

Conversion of NAG to NAM is effected by base-catalysed epimerisation. This reaction may be effected by treating NAG with a base, for example an alkali metal hydroxide such as sodium or potassium hydroxide or a quaternary ammonium ion exchange resin in the hydroxyl form such as Duolite™ A113 (OH⁻), in an aqueous medium, for example water, at ambient or elevated temperature, for example 20 to 50°C, preferably about 25 to 35°C. Epimerisation may be carried out at different concentrations of NAG, but preferably at its saturation concentration. The reaction is continued until equilibration between NAG and NAM is reached. Reaction times will vary with the temperature and pH of the reaction but for example at about 25°C and about pH11 equilibration is achieved at about 20 - 60, for example about 40, hours, or at about 32°C and >pH11 equilibration is achieved at about 12-20, for example about 16, hours. The ratio of NAM to NAG on equilibration is typically about 1:4 by weight.

The NAM/NAG mixture is enriched in NAM by selective removal of NAG. This enrichment may be carried out in a variety of ways. Thus, the NAM/NAG mixture may be concentrated to a residue by evaporation or spray drying. This residue may then be extracted with an organic solvent such as a lower alcohol, preferably methanol, in an amount just sufficient to dissolve the NAM present. Removal of insoluble NAG gives a solution rich in NAM. Alternatively, the NAMINAG mixture, preferably having a high starting concentration of NAG or optionally concentrated to achieve a high concentration of NAG, may be treated with a suitable organic solvent such as a lower alcohol, preferably isopropanol, in the ratio of 1 volume of epimerisation mixture to 5 to 10 volumes, preferably about 6 volumes, of isopropanol, and preferably at a temperature of from 15° to 30°C such as 20° to 25°C. After removal of crystalline NAG the residual solution is rich in NAM. This solution may be further enriched by evaporation of the isopropanol/water azeotrope, preferably with an isopropanol feed to the evaporator, to give a second crop of crystalline NAG. Organic solvent is removed from the NAM-enriched solutions before presenting them to the next step of the process at the preferred concentration in water. By this means the ratio of NAM to NAG may be increased to about 1.5:1 to 10:1, typically, on large scale, 1.5:1 to 4:1. NAG recovered during the enrichment process maybe reused in the epimerisation step, optionally in admixture with fresh NAG.

When a base such as sodium hydroxide is used in the epimerisation step, the reaction mixture is neutralised using, for example, an acid resin [e.g. Amberlite 200 (H⁺) or IR120 (H⁺)] or a suitable acid (e.g. acetic acid) prior to reaction with pyruvate.

In the third step of the process the NAMINAG mixture enriched in NAM is incubated with pyruvate (e.g. sodium pyruvate) in the presence of NANA aldolase, for example Escherichia coli NANA aldolase (EC4.1.3.3).

NANA aldolase is a known enzyme [cf D Comb and S Roseman, J Biol Chem 235, 2529-2537 (1990)] and may be obtained, for example, either from a naturally occurring NANA aldolase-producing strain of E.coli (including constitutive mutant strains) or from an overexpressing recombinant strain of E.coli. Preferably the NANA aldolase is obtained from an over-expressing recombinant strain of E. coli. In particular the NANA aldolase is obtained from either of the recombinant E. coli strains TG1 [pMexAld] and TG1 [pPLAld], described hereinafter and forming further aspects of the present invention.

The NANA aldolase may be used in free form or, preferably, immobilised to allow reuse of the enzyme. Immobilisation may be carried out for example by mixing untreated cell homogenate or partially purified extracts of a NANA aldolase-producing strain in a suitable buffer with an activated resin such as EupergitC™ (Rohm Pharma) and either stirring or standing with occasional mixing for 2 to 15 days. The immobilised beads can then be recovered after use in the reaction by filtration and re-usod. Other immobilisation procedures known in the art can also be utilised, including immobilisation of the enzyme by containment within a hollow fibre or membrane reactor.

The conversion of NAM to NANA is carried out in aqueous medium at a pH of 6.0 to 9.0, for example 7 to 8, at a temperature of 5° to 60 C, for example about 20° to 30°C, for from about 2 to 48 hours. The reaction is terminated when a substantial amount of NAM, up to about 95%, is convened to NANA.

Use of NAM-enriched NAM/NAG mixtures allowing for higher concentrations of NAM offers a number of advantages for NANA production, including a faster reaction, increased productivity and a lower requirement of pyruvate. Most significantly, this results in a high concentration of NANA and low concentrations of NAG and pyruvate which greatly simplifies the isolation process.

When an enriched NAM/NAG mixture is used at high concentration the pyruvate: NAM starting molar ratio should be 1.5 to 2.5:1.

In the fourth step of the process of this invention, the NANA may be isolated from the reaction mixture by crystallisation. Where an enriched NAM/NAG mixture has been used at high concentration with a pyruvate: NAM starting molar ratio of about 1.5 - 2.5:1 and concentration in the reaction mixture is (or can be concentrated to) about 150g/litre then simple crystallisation from the reaction mixture, e.g. by addition of from 4 to 8, preferably 6, volumes of acetic acid, is effective.

When the NANA concentration in the reaction mixture is below 150g/litre, it may be necessary to remove other components from the reaction mixture before NANA is itself isolated.

Sodium ions may conveniently be removed by passing the reaction mixture through an ion exchange resin such as Amberlite™ 200 (H⁺) or IR120 (H⁺). This reduces the pH of the reaction mixture to about 2.

Pyruvate may be selectively removed from the mixture after reaction with metabisulphite anion by absorption of the hydroxysulphonic acid formed onto an ion exchange resin. The reaction and adsorption may be conveniently carried out in one stage by passage of the acidified mixture through a column of quaternary ammonium ion exchange resin such as Duolite™ A113 in the bisulphite form. Any leached bisulphite ions in the effluent from this column may be precipitated by addition of calcium ions.

NAG may be separated from NANA by retention of NANA on a suitable ion exchange resin e.g. Duolite™ A113 in the acetate form. NAG passes through the resin and may be recycled through the process after equilibration to a NAG/NAM mixture as above. The NANA may be eluted from the column with a suitable acid or salt such as formic acid or preferably sodium acetate. NANA may be crystallised from the effluent after suitable concentration by addition of a cosolvent such as acetonitrile or preferably acetic acid. In the case of salt elution of NANA from the ion exchange column it may be preferable to remove cations before any concentration and crystallisation by treatment of the solution with an ion exchange resin in the acid form e.g. Amberlite™ 200 (H⁺) or IR120 (H⁺) as above.

Crystalline NANA produced by these processes may be further treated in a number of ways. The level of solvent in the product may be reduced by drying at an elevated temperature e.g. 30° to 60° in vacuo. Alternatively, the crystalline material may be slurried in acetone containing a small amount of water (eg about 0.5%) and the crystals recovered by filtration and dried as before. The needle crystals produced by the acetic acid crystallisation may be converted either with or without prior desolvation to rhomboid crystals by adding slowly to water (eg about 1 weight per volume). After stirring for an appropriate period (eg about 30min) the crystallisation can be enhanced by the addition of acetone (eg about 8 to 9 volumes per weight initial crystals). The rhomboid crystals can be recovered by filtration, washed with acetone and dried as before. If required the rhomboid crystals can be recrystallised by repeating this procedure. NANA can thus be isolated in highly pure form.

The preparation of overexpressing recombinant E. coli NANA aldolase producing strains is described in Example 1 hereinafter.

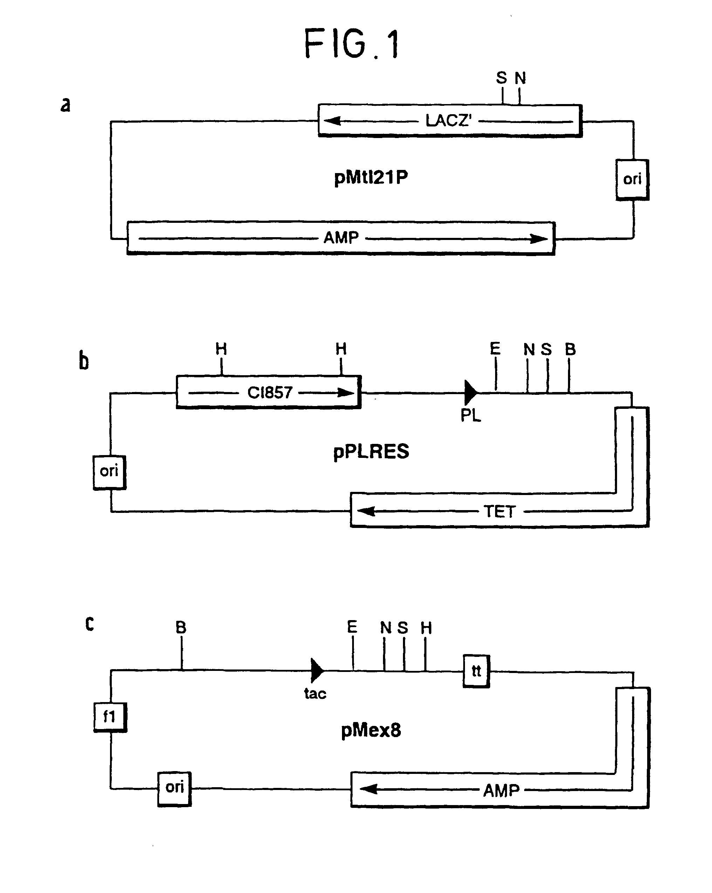

In the Figures :-
Figure 1 shows Maps of Plasmids
   a) pMtl21 P contains a multiple cloning site within the lacZ' gene for which only the Ncol and Sall sites are indicated).
   b) pPLRES contains the bacteriophage lambda phage leftward promoter (PL) and the bacteriophage lambda phage C₁₈₅₇ repressor gene. The gene product from C₁₈₅₇ is a temperature sensitive repressor of the PL promoter. Transcription from PL is induced by a shift from the permissive temperature to the non permissive temperature (usually 30°C to 40-42°C).
   c) pMexB contains the hybrid tac promoter which can be repressed by the product of a host cell encoded lacl gene. Repression is removed by the presence of IPTG. This plasmid also contains the rmB transcription terminator (tt) adjacent to the tac promoter. In addition to the ColE1 origin of replication this plasmid contains the F1-intergenic region (f1).

   AMP, β-lactamase gene; TET, tetracycline resistance gene; ori, origin of replication; B, BamHI; E, EcoRI; H, HindIII; N, Ncol; S, Sall. Maps are not drawn to scale.
Figure 2 shows the sequence of Plasmid pPLRES.
   The sequence of one strand of plasmid pPLRES is shown, starting at the first A of the unique EcoRI site (GAATTC) and proceeding in a rightward direction (5' to 3') around the plasmid as represented in Figure 1b. The circular plasmid is written as a linear sequence.
Figure 3 shows maps of Plasmids containing the Aldolase sequence
   a) pMtlAld.
   b) pMexAld
   c) pPLAJd

   AMP; β-lactamase gene; TET, tetracycline resistance gene; ori, origin of replication f1, f1-intergenic region; tt, rrnB transcription terminator; triangle, promoter used to direct transcription of the aldolase sequence; tac, hybrid tac promoter; PL, bacteriophage lambda phage leftward promoter; C1857, bacteriophage lambda phage temperature sensitive repressor gene; B, BamHI; E, EcoRI; H, Hind III; N, Ncol; S, SaIl. Maps are not drawn to scale.

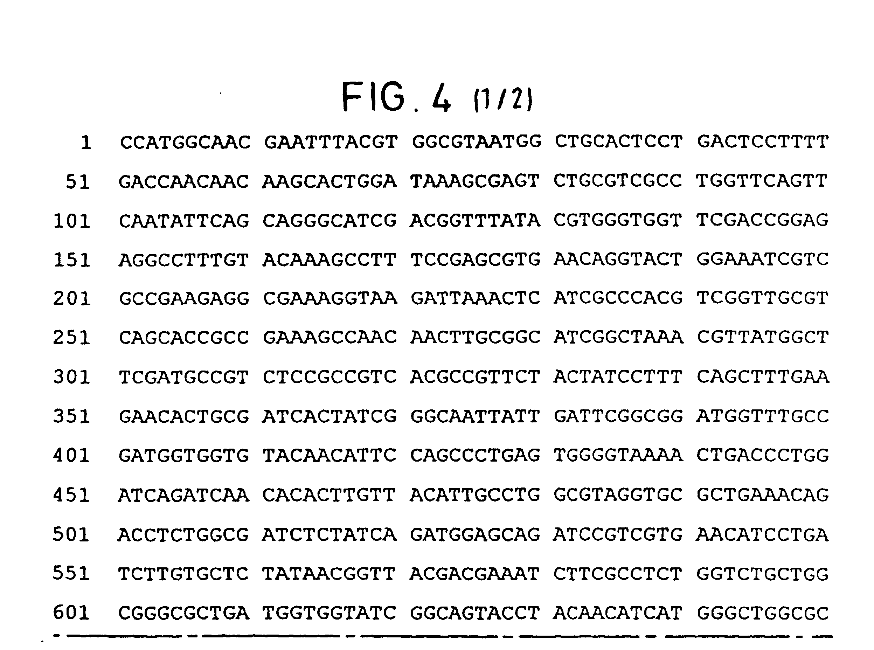

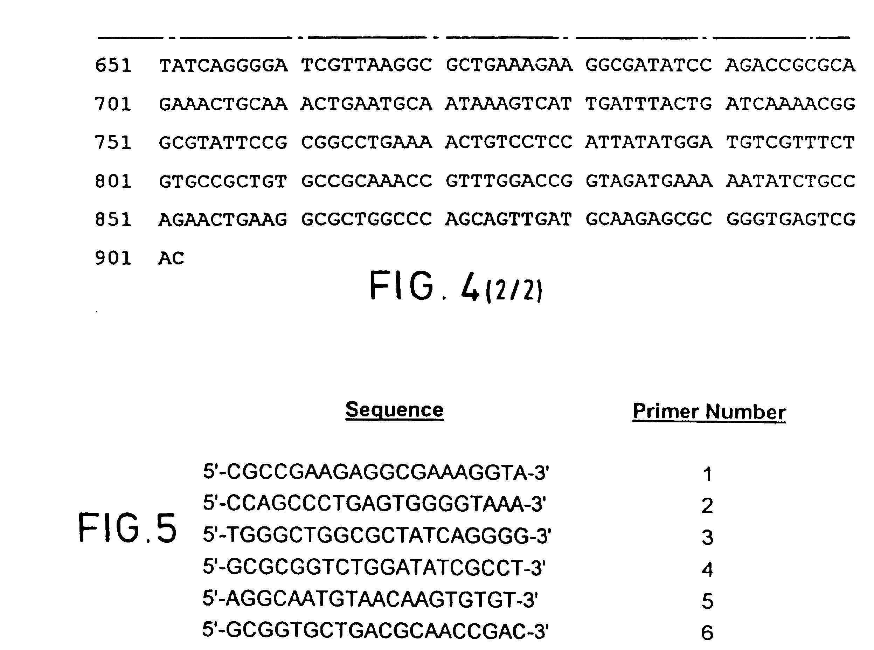

Figure 4 shows the sequence of the cloned Aldolase fragment The sequence includes the Ncol site (CCATGG) and the Sall site (GTCGAC) flanking the aldolase encoding sequence. The coding strand only is shown. The translation initiation codon (ATG) is located at bases 3 to 5.
Figure 5 shows Aldolase sequencing primers

Six synthetic DNA primers were synthesised, based on the published aldolase sequence (Y Ohta et al, Nucleic Acids Research 1985, 13, 8843-8852) and used to sequence regions of the cloned aldolase gene.

The invention is illustrated by the following examples which are not intended as a limitation thereof. All temperatures are in °C.

### Example 1 E. ColiTG1[pMexAld] and E. ColiTG1[pPLAld]

### 1. Materials

X-Gal (5-bromo-4-chloro-3-indolyl-β-D-galactopyranoside) and enzymes Ncol, Sall, T4DNA ligase were obtained from Boehringer Mannheim UK (Diagnostics and Biochemicals). Taq polymerase was obtained from Promega Corporation UK. Geneclean was obtained from Bio 101, Inc. La Jolla California USA. IPTG isopropyl-β-D-thiogalactopyran-oside) was obtained from United States Biochemical. dATP, dCTP dGTP and dTTP, M13mp18 and M13mp19 were obtained from Pharmacia. [α³³P] dATP and [α³⁵S] dATP were obtained from Amersham International PLC.

### 2. Plasmids and E.coli Strains

### a) pMtl21 P

Obtained from Dr NP Minton [ref Chambers SP, Prior SE, Barstow DA, Minton NP, 1988 Gene 68 139-149].

### b) pMex8

Obtained from MEDAC. Gesellshaft fur Klinische Speziälpraparate mbfl Fehlandtstrasse 3. D-2000 Hamburg 36.

### c) pPLRES

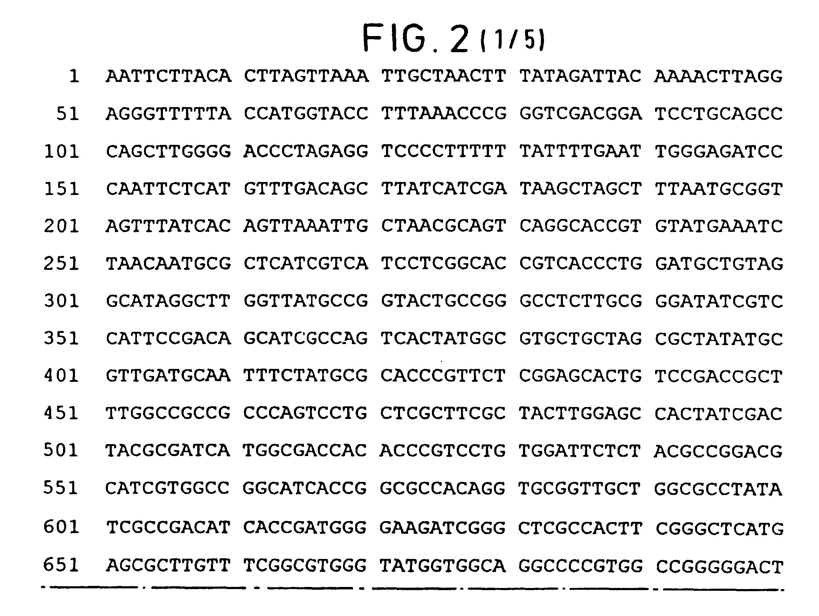

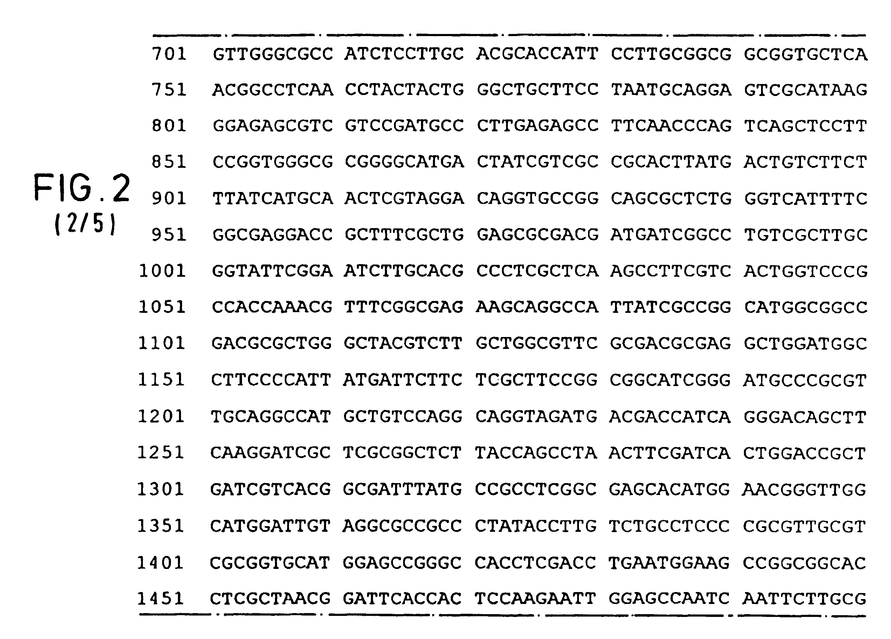

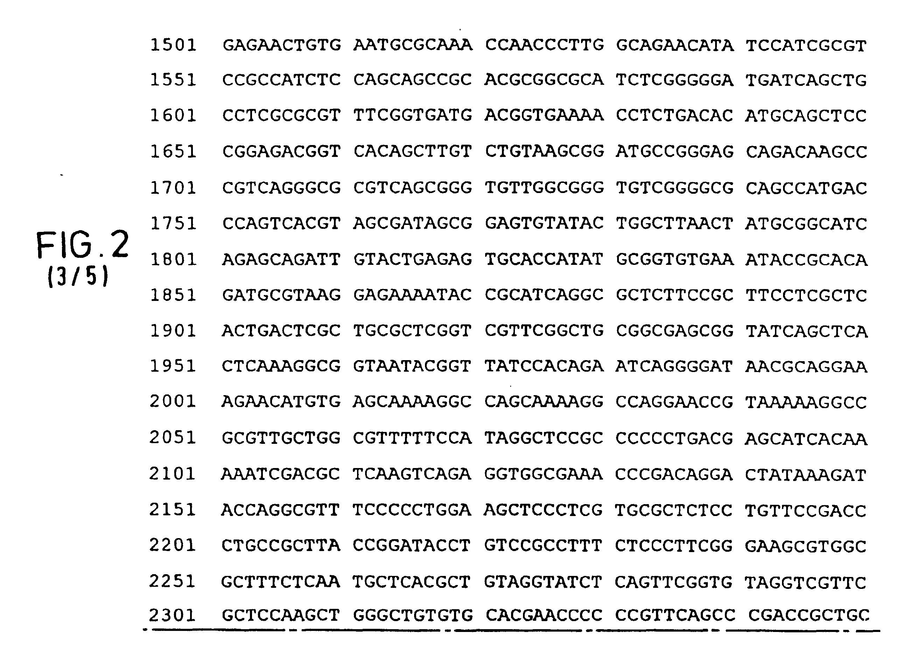

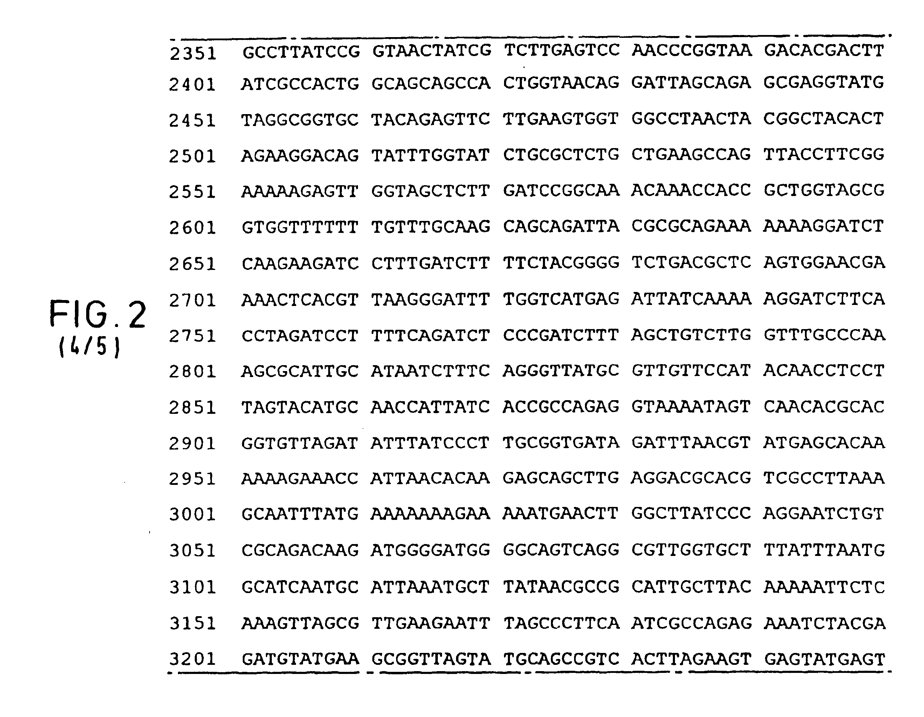

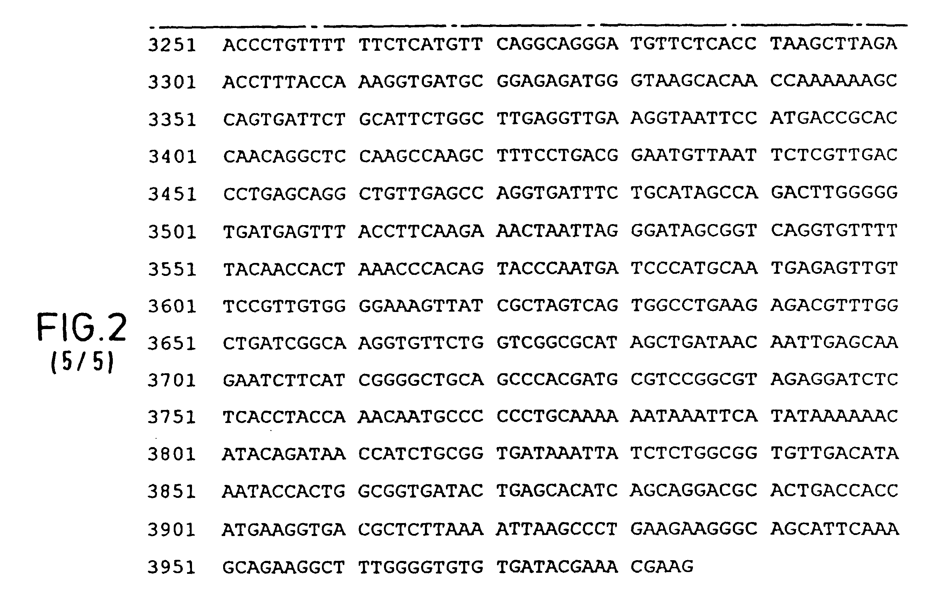

pPLRES was constructed with the sequence shown in Figure 2.

### d) E.coli TGI

Obtained from Amersham International plc. Geneotype: K12 Δ (lac-pro), supE, thi, hsd D5, F' tra D36, proA⁺B⁺, lacl^{q}, lacZΔMl5.

### e) E.coli C600

F ⁻e14 (mcrA⁻) hr-1, leuB2, thi-1, lacY, supE44, rfbD1, fhuA21.

### Growth of Strains

| | | |
|---|---|---|
| C600 | LB | 37°C |
| TG1 | MGT | 37°C |
| TG1/pMex8 | MGT amp | 37°C |
| TG1/pMexAld | MGT amp | 37°C |
| TG1/pPLcddPCR | LBtet | 30°C |
| TG1/pPLAld | LBtet | 30°C |
| TG1pMtl21P | LBamp | 37°C |

### 3. Media

| **LB (per litre)** | |
|---|---|
| NaCl | 10g |
| Yeast extract | 5g |
| Bactotryptone | 10g |
| pH 7.0 | |

### Minimal Salts (per litre)

| | |
|---|---|
| K₂HPO₄ | 7g |
| KH₂PO₄ | 3g |
| MgSO₄.7H₂O | 0.25g |
| (NH₄)₂SO₄ | 1g |
| Tri-sodium citrate | 0.5g |
| pH 7.0 | |

### MGT

Minimal salts supplemented with:
0.4% w/v glucose
0002mg/ml thiamine HC

### MGTC

MGT supplemented with 100ml/litre of 20% w/v casamino acids solution LB and MGT agar plates contained media supplemented with 1.5% w/v agar Antibiotic supplements were at final concentration of:
100µg/ml ampicillin (amp)
5µg/ml tetracycline (tet)

### 4. Methods

a) DNA manipulation including ligations and transformation of E.coli strains were performed essentially as described by Maniatis et al. Molecular cloning, Cold Spring Harbour New York 1982. Transformation of pPLRES derived ligations were carried out by the standard procedure except that heat shock was for 5 minutes at 34°C and the incubation of heat shocked cells in non-selective media was at 30°C rather than at 37°C.
b) Restriction endonuclease digestions were performed using conditions recommended by the suppliers.
c) The nucleotide sequence of the cloned aldolase gene was determined by the M13 dideoxy sequencing method using a Sequenase kit and [α³³P]dATP or [α-³³S]dATP.
**d) Purification of DNA Fragments**
   Fragments of DNA were purified after electophoresis through agarose gel by means of Geneclean glass milk as described by the supplier.
**e) Preparation of Synthetic DNA Sequences**
   Synthetic DNA was prepared by means of Biosearch model SAMI (trade mark) DNA synthesiser using b-cyanoethyl phosphoramidites (ND Sinha et al, Nucleic Acid Res., 1984 12, 4539).
**f) Amplification Reaction**
   The amplification reaction contained 1 mg genomic DNA, 1 unit Taq polymerase, 1 x Promega Taq buffer 50pmol each DNA primer, 0.0125mM each of dATP, dCTP, dGTP and dTTP in a 0.1ml final volume. The reaction was carried out in a Techne PHC-3 Thermal cycler. 1 cycle of 90°C, 10 minutes, 50°C 20 minutes then 3 cycles of 50°C, 1 minute, 70 C, 2 minutes, 92°C, 1 minute then 27 cycles of 50° C, 1 minute, 70°C, 2 minutes, 92°C, 10 seconds then 1 cycle of 50°C, 1 minute, 70°C, 5 minutes, then cooled to 4°C.

### 5. Cloning of Aldolase Encoding Sequence from E.coli C600

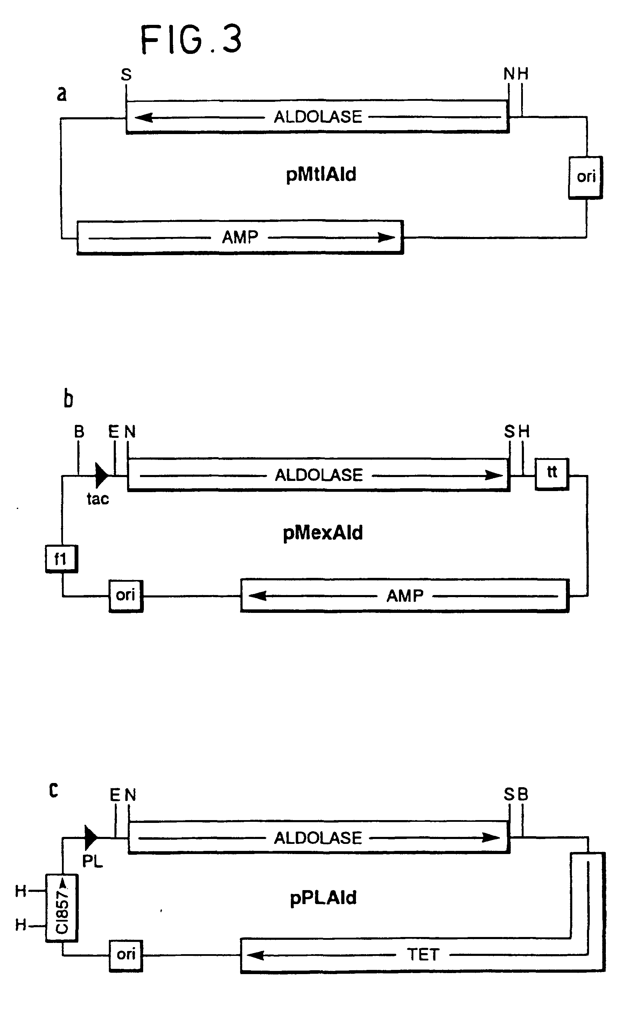

a) Genomic DNA was prepared from 5ml of an ovemight culture of *E.coli* C600, grown in LB at 37°C, by the method described in Current Protocols in Molecular Biology, Voll page 2.4.1 (Eds FM Ausubel *et al*).
b) The aldolase gene was specifically amplified from the E.coli C600 genomic DNA using a Taq polymerase reaction primed by synthetic DNA sequences Ald/PCR/1 and Ald/PCR/2: the design of these primers was based on the published aldolase gene sequence (YOhta *et al*, Nucleic Acids Research, 1985, 13, 8843-8852).
   Ald/PCR/1 
5'-GACGCTACCATGGCAACGAATTTACGTGGCG-3
NcoI
 Ald/PCR/2 
5'-GATCCAGTCGACTCACCCGCGCTCTTGCATC-3
SalI
   The DNA fragment produced by this reaction contained the aldolase encoding sequence flanked by Ncol and Sall sites introduced from the sequence in the primer. The Ncol site (CCATGG) was placed at the position corresponding to the translation initiation codon. The Sall site (GTCGAC) was placed immediately 3' to the termination codon (TGA).
c) The aldolase gene fragment was digested with Ncol and Sall and purified by means of Geneclean™.
   The purified aldolase fragment was ligated into plasmid pMtl21P digested with Ncol and Sall. The ligation reaction was used to transform E.coliTG1 to ampicillin resistance. An ampicillin resistant colony with a white phenotype on LBagar plates supplemented with ampicillin (0.1mglml), Xgal (0.08mg/ml) and IPTG (0.2mM) was isolated that contained the aldolase sequence. The plasmid in this clone was called pMtlAld (Figure 3a).
d) The aldolase fragment from pMtlAid was transferred into M13mp18 and M13mp19 as a BamHI-HindIII fragment and the sequence determined and confirmed to be aldolase by comparison with the sequence published by Y
   Ohta *et al* Nucleic Acids Res. 1985 13, 8843. The sequence of the cloned aldolase fragment in pMtlAld is shown in Figure 4. Primers used to determine the sequence were those shown in Figure 5 and the -40 primer provided with the Sequenase kit.

### 6. Cloning of Aldolase Gene into E.coli Expression Plasmids

a) Plasmid pMtlAld was digested with Ncol and Sall and the aldolase fragment purified by Geneclean™.
b) Plasmid pMex8 was digested with Ncol and Sall and the 3.6 kilobase pair (Kbp) fragment purified by Gene-clean™.
c) Plasmid pPLRES was digested with Ncol and Sall and the 3.96kbp fragment purified by Geneclean™.
d) The aldolase fragment from a) was ligated into the purified pMex8 fragment from b) and the ligation reaction used to transform E.coli TG1 to ampicillin resistance. A clone was isolated that contained the aldolase gene correctly inserted in the pMex8 fragment. The plasmid in this clone was called pMexAld (Figure 3b).
e) The aldolase fragment from a) was ligated into the purified pPLRES fragment from c) and the ligation reaction used to transform *E.coli* TGl to tetracycline resistance. A clone was isolated that contained the aldolase gene correctly inserted into the pPLRES fragment. The plasmid in this clone was called pPLAld (Figure 3c).

### Example 2 - Production of NANA aldolase

0.5ml of a frozen suspension of *E.coli* TG1[pMexAld] was used to inoculate 2 x 400ml of MGTA broth in 2 litre flasks.

| **MGTA** | |
|---|---|
| **INGREDIENT** | **g/L** |
| K₂HPO₄ | 7 |
| KH₂PO₄ | 3 |
| MgSO₄.7H₂O | 0.1 |
| (NH4)₂SO₄ | 1 |
| Tri-sodium citrate | 0.5 |

Distilled water. Natural pH. Sterilise for 121°/15 mins.

Added post-autoclaving:

| | |
|---|---|
| *Thiamine.HCl | 0.002 |
| Glucose | 4 |
| *Ampicillin | 0.05 |

| | |
|---|---|
| *filter sterilised. | |

The ampicillin solution was prepared fresh and added immediately prior to inoculation.

The flasks were incubated overnight at 37°C on a rotary shaker operated at 250 rpm with a 5mm diameter orbital motion. Each flask was used to inoculate 45 litres of MGTCA/1 or MGTCA/2 broth in a 70 litre fermenter.

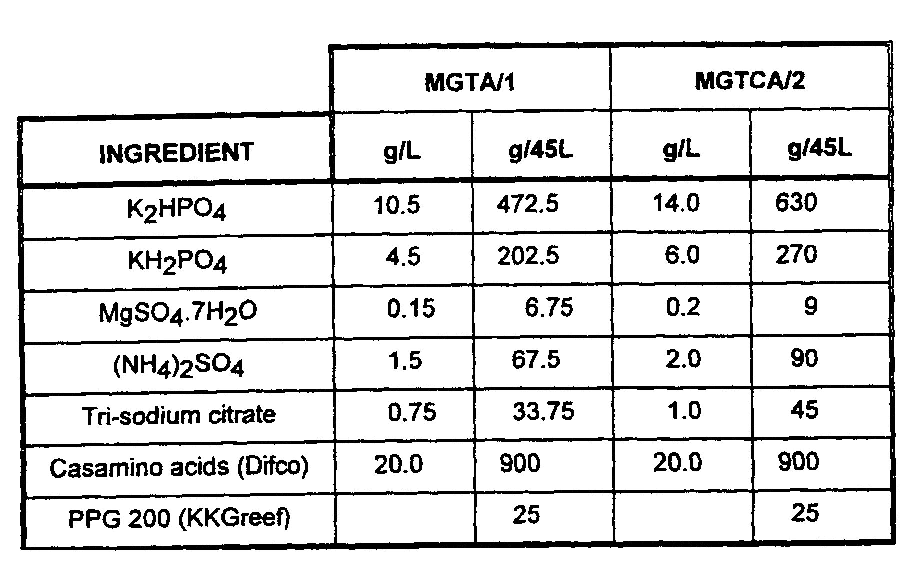

Distilled water. Natural pH. Fermenters were sterilised at 121°C/30 mins.

Added post-autoclaving:

| | | | | |
|---|---|---|---|---|
| Glucose | 6 | 270 | - | - |
| Glycerol | - | - | 8 | 360 |
| *Thiamine. HCI | 0.002 | 0.09 | 0.002 | 0.09 |
| *Ampicillin | 0.05 | 2.25 | 0.05 | 2.25 |

| | | | | |
|---|---|---|---|---|
| *Filter sterilised. The ampicillin solution was prepared fresh and added immediately prior to inoculation. | | | | |

Added two hours after inoculating:-

| | | | | | | |
|---|---|---|---|---|---|---|
| Isopropyl β-D-thio-galactoside (IPTG) | | 0.024 | 1.07 | | 0.024 | 1.07 |
| | (0.1mM) | | | (0.1mM) | | |

On demand:
PPG200 (KKGreef)

The cultures were incubated for 26 hours at 37°C, with stirring at 500 rpm and 451pm airflow. IPTG was added to induce aldolase production 2 hours after inoculation.

The two fermentation broths were bulked and harvested by continuous centrifugation (Sharples) to obtain 1.75kg of wet cell paste. This was suspended in 5.5L of lysis buffer and homogenised by 3 passes through a Manton-Gaulin homogeniser (8000 psi) to obtain the crude extract.

| **LYSIS BUFFER** | |
|---|---|
| **INGREDIENT** | **CONCENTRATION** |
| KH₂PO₄ | 20mM |
| K₂HPO₄ | 20mM |
| ethylenediamine tetra acetic acid (EDTA) | 1mM |
| dithiothreitol (DTT) | 1mm |
| phenylmethanesulphonylfluoride | 1mm |

The aldolase enzyme was immobilised by mixing the crude extract with 1.27kg of Eupergit™-C beads (Rohm Pharma) and allowing the mixture to stand at room temperature with occasional stirring for 10 days. The beads (wet weight 4.44kg) were washed in buffer until the A₂₈₀ Of the supernatant was below 0.1, and stored in the same, but without NaCl, at 4°C.

| **WASH BUFFER (pH 7.0)** | |
|---|---|
| **INGREDIENT** | **CONCENTRATION** |
| Tris-HCI | 100mM |
| NaCl | 500mM |
| EDTA | 1mm |
| DTT | 1mM |
| p-hydroxybenzoic acid ethyl ester | 3mM |

### Example 3 - Production of NANA

### (i) Preparation of NAM-enriched Mixtures

A solution of NAG (1.75kg) and sodium hydroxide (38g) in water (4.37L) was incubated at 25° for 40 hr. Amberlite™ 200 (H⁺) resin (800ml) was added to reduce the pH below 7 and the resin removed by filtration. 2 x 1 L aliquots of the resulting filtrate were treated as follows:
a) A 1L portion was evaporated to give an oily solid. This was stirred with methanol (660ml) at room temperature until a fine white solid remained. This was removed by filtration and washed with methanol (100ml) to give, after drying *in vacuo* at 60°, 194.8g of NAG. The filtrate was evaporated to give an oily solid (91.1g after drying) which was dissolved in water (180ml). This solution was assayed by ion-exclusion HPLC (cf. Kragl, Gygax, Ghisalba and Wandrey in Angew. Chem. Int. Ed. Eng. Vol.30, 1991, p827-828) and found to contain 205g/L NAM, 85g/L NAG (NAM:NAG 2.5:1).
b) The second 1L portion was mixed with isopropanol (5L) and left at room temperature for 3 days. The resulting crystals were filtered off (paper), washed with isopropanol (200ml) and dried *in vacuo at* 60*°* to give NAG (159g). The mother liquors were evaporated to an oily solid which was extracted with methanol (660ml) at room temperature. The residual solid was filtered off and dried in *vacuo* at 60° (56.4g). This solid was found still to contain NAM (NAG:NAM c 3:1) so was reextracted with methanol to give, after drying, NAG (41.6g). The filtrate and wash were combined and evaporated to give an oily solid (14.8g) which was very rich in NAM. This was combined with the oily residue obtained by evaporation of the first filtrate and washes (80.3g) by dissolving in 167ml of water. Assay of this solution by ion-exclusion HPLC gave 224g/L NAM, 66g/L NAG (NAM:NAG 3.4:1).

### (ii) Aldolase Reaction

To 20ml of the NAM-enriched mixture from method b) (4.48g NAM, 1.32g NAG) was added sodium pyruvate (4.54g, 2:1 molar ratio pyruvate:NAM) and enzyme beads (8g wet weight, from Example 2). The reaction mixture was incubated with stirring at 20° for 48h.

### (iii) Isolation of NANA

At the end of the reaction, the reaction mixture was filtered to remove the enzyme beads and the filtrate (20ml) was passed through an Amberlite™ 200 (H⁺) column (40ml). NANA was displaced with 1bv of distilled water, but a further 25ml was needed to achieve good recovery. The eluate was evaporated back to 20ml. Acetic acid (5 volumes) was added and the mixture was left at 4°C for 3 days to crystallise. Needle crystals were removed by filtration and dried *in vacuo* at 60° to give 3.8g solid, assaying 95% NANA.

### Example 4 - Production of NANA

### Epimerisation and Preparation of NAM-enriched mixtures

5kg NAG and 110g sodium hydroxide were dissolved in 12.5 litres of distilled water and the solution allowed to stand at 25°C. After 65 hours 3.3 litres of Amberlite™ 200 (H⁺) (Rohm and Haas) was added to reduce the pH below 7.0 and the resin removed by filtration. The epimerised solution contained NAM and NAG in the approximate ratio of 1:4.

NAG was partially crystallised from the epimerised solution by the addition of propan-2-ol (5 volumes), the solid being recovered after 4 days at 4°C by filtration, and washed with fresh solvent. The propan-2-ol solution was concentrated approximately 6 fold on a Balfors wiped film evaporator giving a second crop of NAG which was recovered by filtration as before. The filtrate was evaporated to dryness on a Buchi rotary evaporator and extracted with a total of 14 litres of methanol. A further crop of NAG was removed at this time. The methanol solution was evaporated to dryness as before, the solid obtained was dissolved to 4.17 litres in water and insoluble material removed by filtration. This solution had a NAM to NAG ratio of approximately 4.5:1.

### Bioconversion/isolation

3.5 litres of the epimerised NAM-enriched mixture (620g NAM, 133g NAG) and 442g of sodium pyruvate were added to a 7 litre vessel. The pH was adjusted to 7.5 with sodium hydroxide and the reaction started by the addition of 1.7kg of washed immobilised enzyme beads from Example 2. The reaction mixture was stirred at 430rpm with a single marine impeller, and the temperature maintained at 20°C. The reaction was allowed to proceed for 46 hours after which the enzyme beads were removed from the reaction mixture by vacuum filtration through filter cloth and washed with 1L distilled water.

NANA was crystallised from the filtrate by the addition of 5 volumes of glacial acetic acid (22.5L). The solid was recovered after 5 days at 4°C by vacuum filtration through Whatman™ 54 paper and washed with fresh acetic acid (4L). Excess acetic acid was removed from the solid by washing with acetone (24L) and the solid dried to constant weight at 50°C in a vacuum oven. Final weight 576.4g, purity by HPLC 96.4%.

### Example 5 - Production of NANA

### (i) Epimerisation and Preparation of NAM-enriched mixtures

Sodium hydroxide pellets (5.0g) were dissolved in process water (1250 ml), NAG (500g) added and the suspension stirred until solution was obtained. The solution was allowed to stand at 33°C for 15 hours when the NAM/NAG ratio was 0.21. The solution was neutralised with glacial acetic acid and then concentrated to 986 ml. Six volumes of isopropanol was added and the suspension stirred for 6 hours. Crystallised NAG was filtered off, washed with isopropanol (300 ml) and dried to give 342g NAG.

### (ii) Bioconversion

The combined filtrate and wash (6.30L) was concentrated to 950 ml, and additional process water added to chase off residual isopropanol. The final concentrate (935 ml) was treated with 86.8g sodium pyruvate and the pH adjusted to 7.4 with glacial acetic acid. NANA aldolase (40% w/v) was added and the suspension stirred for 25¹/₂ hours at 25°.

### (iii) Isolation

The enzyme was filtered off and washed with 2 x 750 ml process water. The filtrate and washes (1.74L) were concentrated to 395 ml, to which was added with swirling 2370 ml of glacial acetic acid and a small quantity of seed. The crystallised suspension was allowed to stand for 7.5 hours at 21°C and then filtered. The cake was washed with 118 ml of 85% acetic acid 15% water followed by isopropanol (590 ml) and dried in vacuo at 50°C. The dried solid (90.9g) assayed at 96.8%.

### Example 6 - Production of NANA aldolase

*E. coli* TG1 [pmexAld] from a freeze dried ampoule was rehydrated using nutrient broth (Oxoid), streaked onto the surface of MGTA agar and incubated at 37°C overnight. Bacteria from areas of the plate having separate colonies were suspended in Brain Heart Infusion broth (Oxoid) containing 10% glycerol and stored in 0.5 ml amounts at -20°C for future use.

A 2 litre flask containing 400 ml of MGTA medium (see Example 2) was inoculated with 0.5 ml of stored (-20°C) suspension and incubated overnight at 37°C on an orbital shaker (50 mm throw) rotating at 250 rpm. The contents of the flask were used to inoculate a fermenter containing 4.5 L of MGTA. The culture in the fermenter was incubated at 37°C with aeration at 4.5 Umin and stirring at 500 rpm until a sharp fall in exhaust CO₂ concentration indicated the end of logarithmic growth (4.5 - 6 hours). The contents of the fermenter were used to inoculate a larger vessel containing 450L of LPSG1 medium maintained at 37°C with stirring at 350 rpm. The air flow to the vessel was set at 450 Umin and when the exhaust CO₂ concentration reached 2% (5 - 5.5 hr) a solution containing 10.7 g of IPTG (isopropyl β-Nthiogalactoside) was pumped into the fermenter. The culture was harvested 18 hours after inoculation.

| **LPSG1 Medium** | |
|---|---|
| **Ingredients** | **g/L** |
| Lab Lemco Powder (Oxoid) | 40 |
| Peptone L37 (Oxoid) | 40 |
| NaCl | 5 |
| Glycol | 50 |

The cell paste was further treated as described in Example 2.

### Example 7 - Production of NANA aldolase

*E. coil* TG1 [pP_{L}Ald] from a freeze dried ampoule was rehydrated using nutrient broth (Oxoid), streaked onto the surface of nutrient agar (Oxoid) containing 5 mg/L of tetracycline hydrochloride and incubated at 30°C overnight. Bacteria from areas of the plate having separate colonies were suspended in Brain Heart Infusion broth (Oxoid) containing 10% glycerol and stored in 1 ml amounts at -20°C for future use.

A 2 litre flask containing 400 ml of MCGTT medium was inoculated with 0.8 ml of stored (-20°C) suspension and incubated overnight at 30°C on an orbital shaker (50 mm throw) rotating at 250 rpm. The contents of the flask were used to inoculate a fermenter containing 450 L of LPSG1 (see Example 6) medium maintained at 30°C with stirring at 200 rpm and an air flow of 450 Umin. After 9 hours, the fermentation temperature was raised to 42°C to induce aldolase production. The culture was harvested 24 hours after inoculation.

| **MCGTT Medium** | |
|---|---|
| **Ingredients** | **g/L** |
| K₂HPO₄ | 7 |
| K₂HPO₄ | 3 |
| MgSO₄.7H₂O | 0.1 |
| (NH₄)₂SO₄ | 1 |
| Tri-sodium citrate | 0.5 |
| Casamino Acids (Difco) | 20 |

Dissolve in distilled water. Natural pH. Sterilise at 121°C for 15 min.

Added post-autoclaving

| **Solution** | **Conc. (g/L)** | **Sterilisation** | **Volume (ml/L)** | **Final conc. (g/L)** |
|---|---|---|---|---|
| Glucose | 400 | 121°C 15 min | 10 | 4 |
| Thiamine.HCL | 1 | Filtration | 2 | 0.002 |
| Tetracycline.HCL | 2.5 | Filtration | 2 | 0.005 |

Freshly-prepared tetracycline solutions were added to the medium immediately before inoculation.

The cell paste was further treated as described in Example 2.

### Example 8 Production of NANA

### (i) Epimersation

Sodium hydroxide pellets (1.96g) were dissolved in water (490ml), NAG (196g 7th cycle of use) was added and the suspension stirred until solution was obtained. The solution was allowed to stand at 33°C for 19 hours when the NAM/NAG ratio was 0.21. The solution was neutralised with glacial acetic acid.

### (ii) NAM/NAG Enrichment

The solution was concentrated to 380 mls (0.63 of its original volume). Six volumes of isopropanol (2280 ml) were added and the suspension stirred for 5.5 hours (NAM/NAG ratio 2.14). Crystallised NAG was filtered off, and washed with isopropanol (196ml) and dried in a vacuum oven at 50°C overnight to give NAG (130.1g).

### (iii) Bioconversion

The combined filtrate and wash (2660 ml) from NAG filtration was concentrated to 345 ml, and additional process water added to chase off residual isopropanol. The final concentrate (300ml NAM concentration 90.5 g/l) was treated with sodium pyruvate (27.12g) (2.15 M sodium pyruvate : 1 M NAM) and the pH adjusted to 7.4 with glacial acetic acid. NANA aldolase was added and the suspension stirred for 24 hours at 25°C, this resulting in a conversion of 90%.

### (iv) Isolation

The enzyme was filtered off and washed with water (240ml). The filtrate and washes (590ml) were concentrated to 136 ml, to which was added with mixing 816 ml (6 vols) of glacial acetic acid and a small quantity of seed. The crystallised suspension was allowed to stand for 17 hours at 21°C and then filtered. The cake was washed with 41 ml (0.3 vol of 85% acetic acid 15% water followed by acetone (204 mls - 1.5 vol). The wet cake (62.93g) was then used for subsequent work.

### (v) Acetone Desolvation

Wet cake (30g) was added to 0.5% aqueous acetone (268ml) and the suspension was stirred at room temperature (21°C) for 2 hours, and then filtered. The cake was washed with acetone (2 x 40 mls), and dried in a vacuum oven at 50°C overnight to give desolvated NANA (12.44g) (Potency 99.2%, corrected for water content).

### (vi) Rhomboid Crystallisation

Wet cake (30g) was added slowly to water (27 mls) whilst stirring. The suspension was stirred for 30 minutes at 21°C, by which time the wet cake had converted to rhomboids. Acetone (240 mls) was added over 30 minutes. After acetone addition the mixture was stirred for 1.5 hours at 21°C. The mixture was then cooled to 5°C for 1 hour and filtered. The cake was washed with acetone (2 x 40mls), and dried in a vacuum oven at 35°C overnight to give NANA rhomboids (13.44g). (Potency 99.4%, corrected for water content).

### Example 9 - Crystallisation of Rhomboid Crystals

N-Acetyl-D-Neuraminic rhomboid crystals (56g) were added to stirred water (100ml) at room temperature over 25 - 30 minutes. Initially solution occurred but rhomboid crystals later precipitated. The slurry was stirred for 30 minutes and acetone (800mls) added over 30 minutes. The slurry was stirred for 2 hours at room temperature. The solids were filtered, washed with a acetone water mixture (8:1, 100ml) followed by acetone (300ml) and dried in vacuo at 35°C ovemight. Yield solid, 94.1% ^{w}/W.

## Claims

1. A process for the preparation of N-acetyl-D-neuraminic acid (NANA), which process comprises the steps of: -
1. converting N-acetyl-D-glucosamine (NAG) to N-acetyl-D-mannosamine (NAM) by base-catalysed epimerisation to yield an equilibrium mixture of NAG/NAM;
2. selectively removing NAG from the equilibrium mixture;
3. reacting NAM with pyruvate in the presence of NANA aldolase; and
4. Isolating the thus-produced NANA, by crystallisation from the reaction mixture;
wherein the pyruvate:NAM starting molar ratio for step 3 is 1.5:1 to 2.5:1 and the concentration of NANA in the final reaction mixture is about 150g/litre.

2. A process as claimed in Claim 1 wherein the reaction mixture is neutralised prior to step 3.

3. A process as claimed in Claim 1 or Claim 2 wherein removal of NAG is effected by treatment of the NAM/NAG mixture with an organic solvent and removal of crystalline NAG.

4. A process as claimed in Claim 3 wherein the organic solvent is a lower alcohol.

5. A process as claimed in Claim 3 or Claim 4 wherein the organic solvent is isopropanol.

6. A process as claimed in Claim 5 wherein the ratio of isopropanol: NAM/NAG mixture is 5:1 to 10:1.

7. A process as claimed in Claim 5 or 6 wherein further enrichment is effected by evaporation of the isopropanol/water azeotrope.

8. A process as claimed in any one of claims 5 to 7 wherein the enrichment is effected at a temperature of from 15° to 30°C.

9. A process as claimed in any one of Claims 1 to 8 wherein crystallisation of NANA from the reaction mixture is effected by addition of from 4 to 8 volumes of acetic acid.

10. A process as claimed in any one of Claims 1 to 9 wherein the NAG removed from the NAM/NAG mixture is reused in step 1 of the process.

11. A process as claimed in any one of Claims 1 to 10 wherein the NANA aldolase is obtained from an over-expressing recombinant strain of E.coli.

12. A process as claimed in Claim 11 wherein the recombinant strain of E.coli is TG1[pMexAld] or TG1[pPLAld].

13. A process as claimed in any one of Claims 1 to 12 wherein the NANA aldolase is immobilised.

14. A process as claimed in any one of Claims 1 to 13 wherein step 3 is effected at pH 6 to 9 and temperature 5° to 60°C.

15. A process as claimed in any of Claims 1 to 14 wherein NANA is isolated as needle - shaped crystals.

16. A process as claimed in any of Claims 1 to 14 wherein NANA is isolated as rhombic crystals.

17. A process for the preparation of rhombic crystals of NANA which process comprises addition of acetone to an aqueous solution of NANA.

## Patentansprüche

1. Verfahren zur Herstellung von N-Acetyl-Dneuraminsäure (NANA), umfassend die Schritte:
1. Umwandlung von N-Acetyl-D-glucosamin (NAG) in N-Acetyl-D-mannosamin (NAM) durch basenkatalysierte Epimerisierung unter Erhalt eines Gleichgewichtsgemisches aus NAG/NAM;
2. selektives Entfernen von NAG aus dem Gleichgewichtsgemisch;
3. Umsetzen von NAM mit Pyruvat in der Gegenwart von NANA-Aldolase; und
4. Isolieren der somit hergestellten NANA durch Kristallisation aus dem Reaktionsgemisch;
wobei das molare Anfangsverhältnis Pyruvat:NAM bei dem Schritt 3 1,5:1 bis 2,5:1 ist, und die Konzentration von NANA im Reaktionsendgemisch ungefähr 150 g/Liter ist.

2. Verfahren nach Anspruch 1, wobei das Reaktionsgemisch vor dem Schritt 3 neutralisiert wird.

3. Verfahren nach Anspruch 1 oder Anspruch 2, wobei das Entfernen von NAG durch Behandlung des NAM/NAG-Gemisches mit einem organischen Lösungsmittel und Entfernen des kristallinen NAG durchgeführt wird.

4. Verfahren nach Anspruch 3, wobei das organische Lösungsmittel ein niederer Alkohol ist.

5. Verfahren nach Anspruch 3 oder Anspruch 4, wobei das organische Lösungsmittel Isopropanol ist.

6. Verfahren nach Anspruch 5, wobei das Verhältnis I-sopropanol:NAM/NAG-Gemisch 5:1 bis 10:1 ist.

7. Verfahren nach Anspruch 5 oder Anspruch 6, wobei die weitere Anreicherung durch die Verdampfung des Isoproanol/Wasser-Azeotrops durchgeführt wird.

8. Verfahren nach einem der Ansprüche 5 bis 7, wobei die Anreicherung bei einer Temperatur von 15° bis 30°C durchgeführt wird.

9. Verfahren nach einem der Ansprüche 1 bis 8, wobei die Kristallisation von NANA aus dem Reaktionsgemisch durch den Zusatz von 4 bis 8 Volumen Essigsäure durchgeführt wird.

10. Verfahren nach einem der Ansprüche 1 bis 9, wobei das aus dem NAM/NAG-Gemisch entfernte NAG in Schritt 1 des Verfahrens wiederverwendet wird.

11. Verfahren nach einem der Ansprüche 1 bis 10, wobei die NANA-Aldolase von einem überexprimierenden rekombinanten E. coli-Stamm erhalten wird.

12. Verfahren nach Anspruch 11, wobei der rekombinante E. coli-Stamm TG1[pMexAld] oder TG1[pPLAld] ist.

13. Verfahren nach einem der Ansprüche 1 bis 12, wobei die NANA-Aldolase immobilisiert wird.

14. Verfahren nach einem der Ansprüche 1 bis 13, wobei der Schritt 3 bei einem pH von 6 bis 9 und einer Temperatur von 5° bis 60°C durchgeführt wird.

15. Verfahren nach einem der Ansprüche 1 bis 14, wobei NANA als nadelförmige Kristalle isoliert wird.

16. Verfahren nach einem der Ansprüche 1 bis 14, wobei NANA als rhombische Kristalle isoliert wird.

17. Verfahren für die Herstellung von rhombischen Kristallen von NANA, umfassend den Zusatz von Aceton zu einer wässrigen Lösung von NANA.

## Revendications

1. Procédé pour la préparation de l'acide N-acétyl-D-neuraminique (NANA), lequel procédé comprend les étapes consistant'à :
1. convertir la N-acétyl-D-glucosamine (NAG) en N-acétyl-D-mannosamine (NAM) par épimérisation catalysée par une base, pour donner un mélange d'équilibre NAG/NAM ;
2. éliminer sélectivement la NAG du mélange d'équilibre ;
3. faire réagir la NAM avec le pyruvate en présence de NANA aldolase ; et
4. isoler le NANA ainsi produit, par cristallisation à partir du mélange réactionnel ;
dans lequel le rapport molaire de départ de pyruvate : NAM pour l'étape 3 varie de 1,5 : 1 à 2,5 : 1, et la concentration en NANA dans le mélange réactionnel final est d'environ 150 g/litre.

2. Procédé selon la revendication 1, dans lequel on neutralise le mélange réactionnel avant d'effectuer l'étape 3.

3. Procédé selon la revendication 1 ou 2, dans lequel on enlève la NAG par traitement du mélange NAM/NAG par un solvant organique, et on enlève la NAG cristalline.

4. Procédé selon la revendication 3, dans lequel le solvant organique est un alcool inférieur.

5. Procédé selon la revendication 3 ou 4, dans lequel le solvant organique est l'isopropanol.

6. Procédé selon la revendication 5, dans lequel le rapport d'isopropanol : mélange NAM/NAG varie de 5 : 1 à 10 : 1.

7. Procédé selon la revendication 5 ou 6, dans lequel on effectue un enrichissement supplémentaire par évaporation de l'azéotrope isopropanol/eau.

8. Procédé selon l'une quelconque des revendications 5 à 7, dans lequel on effectue l'enrichissement à une température de 15° à 30°C.

9. Procédé selon l'une quelconque des revendications 1 à 8, dans lequel on effectue la cristallisation du NANA à partir du mélange réactionnel par l'ajout de 4 à 8 volumes d'acide acétique.

10. Procédé selon l'une quelconque des revendications 1 à 9, dans lequel on réutilise la NAG enlevée du mélange NAM/NAG dans l'étape 1 du procédé.

11. Procédé selon l'une quelconque des revendications 1 à 10, dans lequel la NANA aldolase est obtenue à partir d'une souche de *E. coli* recombinante sur-exprimant.

12. Procédé selon la revendication 11,dans lequel la souche de *E. coli* recombinante est TG1[pMexAld] ou TG1(pPLAld).

13. Procédé selon l'une quelconque des revendications 1 à 12, dans lequel la NANA aldolase est immobilisée.

14. Procédé selon l'une quelconque des revendications 1 à 13, dans lequel on effectue l'étape 3 à un pH de 6 à 9 et à une température de 5 ° à 60°C.

15. Procédé selon l'une quelconque des revendications 1 à 14, dans lequel on isole le NANA sous forme de cristaux aciculaires.

16. Procédé selon l'une quelconque des revendications 1 à 14, dans lequel on isole le NANA sous forme de cristaux rhombiques.

17. Procédé de préparation de cristaux rhombiques de NANA, qui comprend l'ajout d'acétone à une solution aqueuse de NANA.
